# EUROPEAN PATENT APPLICATION

(11) **EP 2 042 214 A1**
(43) Date of publication of application: **01.04.2009**
(21) Application number: 08015422.2
(22) Date of filing: 01.09.2008
(51) Int. Cl.: A61M 39/04, A61M 39/22, A61M 39/28

(54) **Liquid coinfusion device**

(30) Priority: 27.09.2007 JP 2007251369
(71) Applicant: Covidien AG, 8212 Neuhausen am Rheinfall (CH)
(72) Inventor: Kitani, Ichiro, Shizuoka-ken (JP)
(74) Representative: Olsson, Carl H.S.

(57) **Abstract**

The provision of a fluid mixture delivery instrument which can mix two types of fluid and then send them upstream, and which is simple to operate.

A tube 24 is arranged inside a downstream pipe 14 in a fluid mixture delivery instrument A which is provided with an upstream pipe 13, downstream pipe 14 and a merging pipe 12 which are respectively connected to a chamber part 11, and also the downstream pipe 14 is provided with pressure parts 23 for closing off the tube 24 by the application of pressure. Furthermore, two pressure parts 23 are provided, one either side of the tube 24. In addition, the pressure parts 23 are provided at one end of a rocking piece 22, the other end of which is supported outside a fluid mixture delivery instrument main body 10. The downstream end of the tube 24 is then fixed to the inner peripheral surface of the downstream pipe 14 by a fixing member 25.

## Description

The present invention relates to a fluid mixture delivery instrument comprising a channel for sending fluid from an upstream pipe to a downstream pipe via a chamber part, and a channel for sending another fluid from a merging pipe to the downstream pipe via the chamber part.

### [Prior Art]

Conventionally, certain fluids such as physiological saline and drug solutions are supplied inside the body of a patient using a transfusion tube. In such cases, a device for ensuring that the correct amount of fluid is supplied may be used in order to regulate the flow rate of said fluid flowing inside the transfusion tube (see Patent Document 1, for example). This device for ensuring that the correct amount of fluid is supplied is configured by a main body comprising an inner wall of which the lower part is formed in a valve chamber and two pipe joints provided with holes which extend from both sides of the valve chamber towards the outside, respectively, and a mobile portion rotatably arranged inside the inner wall of the main body. The two pipe joints of the main body are then formed with a vertical offset between their respective central axes, and the lower part of the mobile portion forms an oblique surface. Consequently, it is possible to open and close the holes of the two pipe joints using the mobile portion while varying the degree of opening of the holes, by causing the mobile portion to rotate. By these means, the flow rate of fluid sent from one pipe joint to the other pipe joint via the valve chamber can be regulated at will.

[Patent Document 1] Japanese Unexamined Patent Application Publication S62-172962

### [Disclosure of the Invention]

However, the abovementioned device for ensuring that the correct amount of fluid is supplied is used in a transfusion tube for supplying only one type of fluid inside the body, and does not therefore enable the use of a transfusion tube for supplying multiple fluids inside the body. Consequently, a fluid mixture delivery instrument has been developed which can supply multiple fluids to a patient by opening and blocking a plurality of transfusion tubes. However, conventional fluid mixture delivery instruments have a structure in which the desired pipe from among the plurality of pipes is opened or blocked by the rotation of a valve element, and the operation to switch flow channels is therefore troublesome. There are also occasions when the practitioner wishes to cause temporary reverse flow of one fluid to the portion housing another fluid, with the aim of mixing one fluid with the other fluid, but this kind of operation cannot be carried out with a conventional fluid mixture delivery instrument.

The present invention has been devised in view of the situation described above, and it aims to provide a fluid mixture delivery instrument which makes it possible to mix two types of fluid and then to send them downstream, and which is simple to operate.

In order to achieve the abovementioned aim, a structural feature of the fluid mixture delivery instrument pertaining to the present invention lies in the fact that it comprises a fluid mixture delivery instrument main body provided with an upstream pipe for sending fluid to a chamber part, a downstream pipe to which the fluid is sent from the chamber part, and a merging pipe for sending another fluid to the chamber part; a downstream flexible tube which is arranged inside the downstream pipe, and inside which the fluid sent from the chamber part to the downstream pipe flows; and a downstream pressure part which extends from the outside to the inside of the downstream pipe, and which closes off the downstream flexible tube by means of pressure applied to the inside of the downstream pipe.

The fluid mixture delivery instrument pertaining to the present invention which is configured in this way comprises an upstream pipe, a downstream pipe and a merging pipe which respectively link in communication with a chamber part. Furthermore, a downstream flexible tube through which flows fluid sent from the chamber part to the downstream pipe is arranged inside the downstream pipe, and said downstream flexible tube can be opened and closed by the pressure-operation of a downstream pressure part. Consequently, in a state in which the downstream flexible tube is closed off by the pressure-operation of the downstream pressure part, for example, it is possible to send a fluid supplied from a merging pipe to the upstream side of an upstream pipe, and to mix this with a fluid such as a drug solution which is supplied from the upstream pipe by supplying a fluid such as a drug solution from the merging pipe to the chamber part side.

Then, after the two types of fluid have been mixed, the two mixed fluids can be sent from the upstream pipe to the downstream pipe via the chamber part by releasing the pressure on the downstream pressure part to allow both ends of the downstream flexible tube to link in communication. By virtue of this, it is possible to supply two types of fluid simultaneously to the body of the patient, and to supply the two types of fluid after they have been mixed. As a result, it is possible to correctly supply two of any type of fluids to the body of the patient.

Furthermore, a rubber stopper is preferably fitted to the merging pipe to block the channel between the merging pipe and the chamber part, and it is preferably possible to form a channel between the merging pipe and the chamber part by piercing the rubber stopper with a cylindrical needle or syringe etc. This makes it possible to open and block the portion between the chamber part and the downstream pipe using the downstream flexible tube, aside from which it is possible to block and open the flow channel running from the merging pipe to the chamber part. Furthermore, the downstream flexible tube is preferably made of an elastic material which returns to its original cylindrical shape, but it may also be made of a material which lacks elastic return force. In this case, when the portions of the downstream pressure part to which pressure is applied come into pressure contact with one another and close, and the pressure is then released, the downstream flexible tube becomes a cylindrical shape in which both ends are linked in communication due to the flow of fluid.

A further structural feature of the fluid mixture delivery instrument pertaining to the present invention lies in the fact that two downstream pressure parts are provided, one on either side of the downstream flexible tube. By virtue of this, the operation to apply pressure to the downstream pressure parts can be easily achieved, simply by pinching and pushing both downstream pressure parts between the thumb and forefinger.

Another further structural feature of the fluid mixture delivery instrument pertaining to the present invention lies in the fact that the downstream pressure part is provided at one end of a downstream rocking piece, the other end of which is supported outside the fluid mixture delivery instrument main body. This makes it possible, when the downstream pressure part is pressure-operated, for the fingers to make contact with the downstream rocking piece instead of the downstream pressure part. This then makes it possible to increase the surface area of the portion which the fingers make contact with on the downstream rocking piece, and therefore the operation to apply pressure to the downstream rocking piece is simplified. Furthermore, the downstream rocking piece preferably normally lies in a position which maintains a certain spacing from the outer surface of the fluid mixture delivery instrument main body, and is preferably configured by an elastic body which bends to the fluid mixture delivery instrument main body side only when it is pressure-operated.

Another further structural feature of the fluid mixture delivery instrument pertaining to the present invention lies in the fact that the upstream end or the downstream end of the downstream flexible tube is fixed to the inner peripheral surface of the downstream pipe or to the inside of the chamber part, by a cylindrical fixing member. This makes it possible to securely fix the downstream flexible tube to the inner peripheral surface of the upstream pipe or to the inside of the chamber part. It should be noted that when the downstream end of the downstream flexible tube is fixed to the inner peripheral surface of the downstream pipe by a fixing member, the downstream flexible tube is deformed by the pressure of the downstream pressure part, but when the pressure is released it returns to its original shape, and it is made of a material which is not deformed by the flow of the fluid.

Another further structural feature of the fluid mixture delivery instrument pertaining to the present invention lies in the fact that it comprises an upstream flexible tube which is arranged inside the upstream pipe, and inside which the fluid sent from the upstream pipe to the chamber part flows; and an upstream pressure part which extends from the outside to the inside of the upstream pipe, and which closes off the upstream flexible tube by means of pressure applied to the inside of the upstream pipe.

With the fluid mixture delivery instrument of the present invention configured in the manner described above, in addition to the fact that the downstream pipe is provided with a downstream flexible tube, an upstream flexible tube through which fluid sent from the upstream pipe to the chamber part is caused to flow, is also arranged inside the upstream pipe. It is then possible to close off said upstream flexible tube by means of the pressure-operation of the upstream pressure part. Consequently, it is possible to stop and reopen the supply of both or either of the two types of fluid sent from the upstream pipe to the downstream pipe via the chamber part by applying pressure to the upstream pressure part or releasing the pressure therefrom. This makes it possible to supply any one or two types of fluid to the patient's body.

Furthermore, in a state in which the upstream pressure part is pressure-operated to close off the upstream flexible tube, if another fluid is supplied from the merging pipe side to the downstream pipe side, via the chamber part, said fluid flows to the downstream side without flowing back to the upstream pipe side. Consequently, in a state in which the upstream flexible tube is closed off, if the required amount of the other fluid is supplied to the downstream pipe side from the merging pipe side, and afterwards the pressure is released from the upstream pressure part, both end openings of the upstream flexible tube are linked in communication so that the first fluid flows from the upstream pipe side to the downstream pipe side, and it flows to the patient's body together with the other fluid which is supplied from the merging pipe side. By virtue of this, the desired fluid(s) is(are) correctly supplied to the patient's body. Furthermore, the upstream flexible tube is preferably made from the same material as the downstream flexible tube.

Another further structural feature of the fluid mixture delivery instrument pertaining to the present invention lies in the fact that two upstream pressure parts are provided, one on either side of the upstream flexible tube. By virtue of this, the operation to apply pressure to the upstream pressure parts can be easily achieved, simply by pinching and pushing both pressure parts between the thumb and forefinger.

Another further structural feature of the fluid mixture delivery instrument pertaining to the present invention lies in the fact that the upstream pressure part is provided at one end of an upstream rocking piece, the other end of which is supported outside the fluid mixture delivery instrument main body. This makes it possible, when the upstream pressure part is pressure-operated, for the fingers to make contact with the upstream rocking piece instead of the upstream pressure part. This then makes it possible to increase the surface area of the portion which the fingers make contact with on the upstream rocking piece, and therefore the operation to apply pressure to the upstream rocking piece is simplified. Furthermore, the upstream rocking piece preferably normally lies in a position which maintains a certain spacing from the outer surface of the fluid mixture delivery main body, and is preferably configured by an elastic body which bends to the fluid mixture delivery instrument main body side only when it is pressure-operated.

Another further structural feature of the fluid mixture delivery instrument pertaining to the present invention lies in the fact that the upstream end of the upstream flexible tube is fixed to the inner peripheral surface of the upstream pipe by a cylindrical fixing member. This makes it possible to securely fix the upstream flexible tube to the inner peripheral surface of the upstream pipe.

Another further structural feature of the fluid mixture delivery instrument pertaining to the present invention lies in the fact that it comprises: a fluid mixture delivery instrument main body provided with an upstream pipe for sending fluid to a chamber part, a downstream pipe to which the fluid is sent from the chamber part, and a merging pipe for sending another fluid to the chamber part; a downstream flexible tube which is arranged inside the downstream pipe, and inside which the fluid sent from the chamber part to the downstream pipe flows; an upstream flexible tube which is arranged inside the upstream pipe, and inside which the fluid sent from the upstream pipe to the chamber part flows; a downstream pressure part which extends from the outside to the inside of the downstream pipe, and which closes off the downstream flexible tube by means of pressure applied to the inside of the downstream pipe; an upstream pressure part which extends from the outside to the inside of the upstream pipe, and which closes off the upstream flexible tube by means of pressure applied to the inside of the upstream pipe; and a rocking piece at both ends of which provision is made for the downstream pressure part and the upstream pressure part, and the central part of which is supported outside the fluid mixture delivery instrument main body so as to be able to rock back and forth.

By virtue of this, it is possible to send fluid supplied from the merging pipe to the upstream side of the upstream pipe, and to mix this with fluid which is supplied from the upstream pipe, by applying pressure to the downstream pressure part to close off the downstream flexible tube. Furthermore, it is possible to stop and reopen the supply of both or either of the two types of fluid sent from the upstream pipe to the downstream pipe via the chamber part by applying pressure to the upstream pressure part or releasing the pressure therefrom. The pressure-operation of the downstream pressure part at this time then makes it possible to close off the downstream flexible tube which is provided in the downstream pipe by means of the pressure applied to the downstream pressure part portion of the rocking piece, while the pressure-operation of the upstream pressure part makes it possible to close off the upstream flexible tube provided in the upstream pipe by means of the pressure applied to the upstream pressure part portion of the rocking piece, and these are extremely simple operations.

### [Optimum Mode of Embodiment of the Invention]

### (Mode of Embodiment 1)

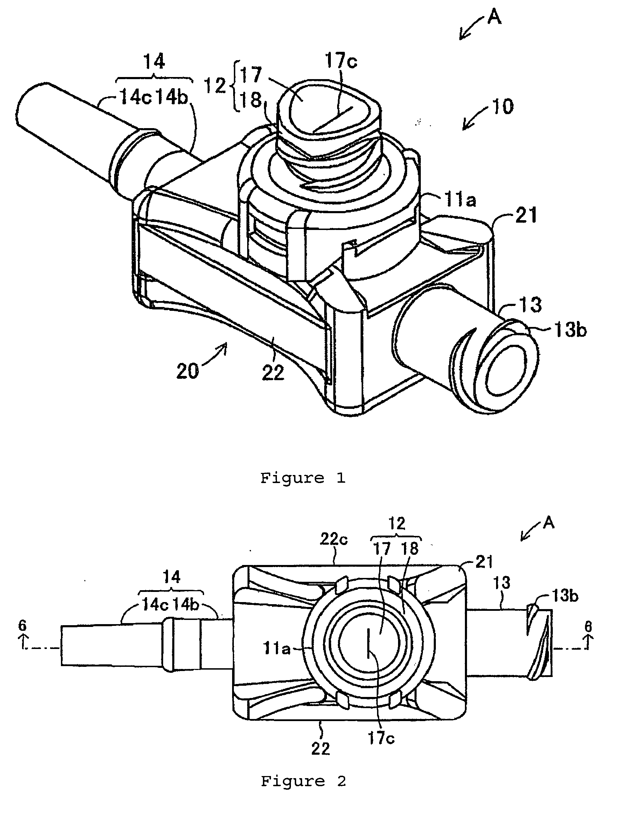

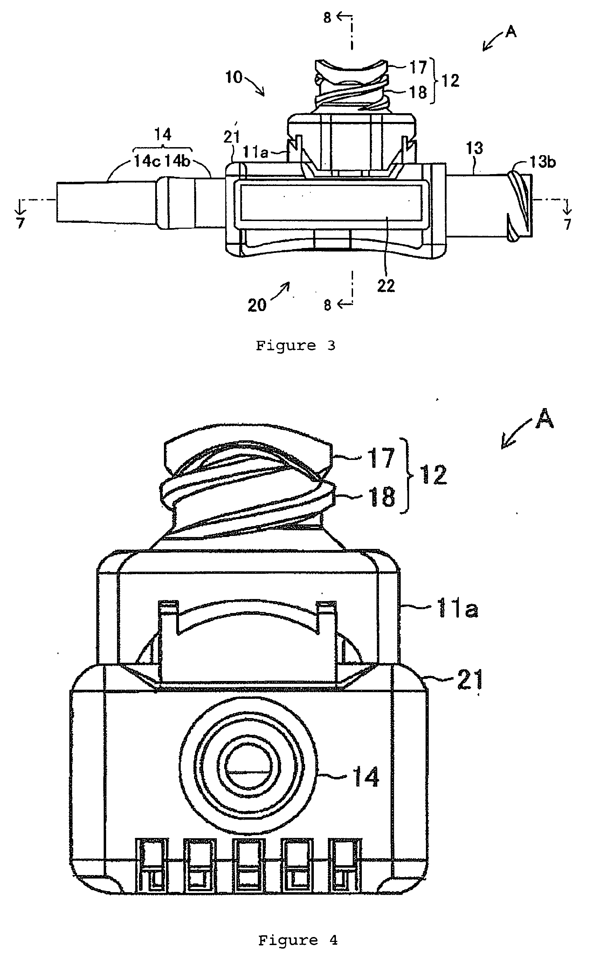

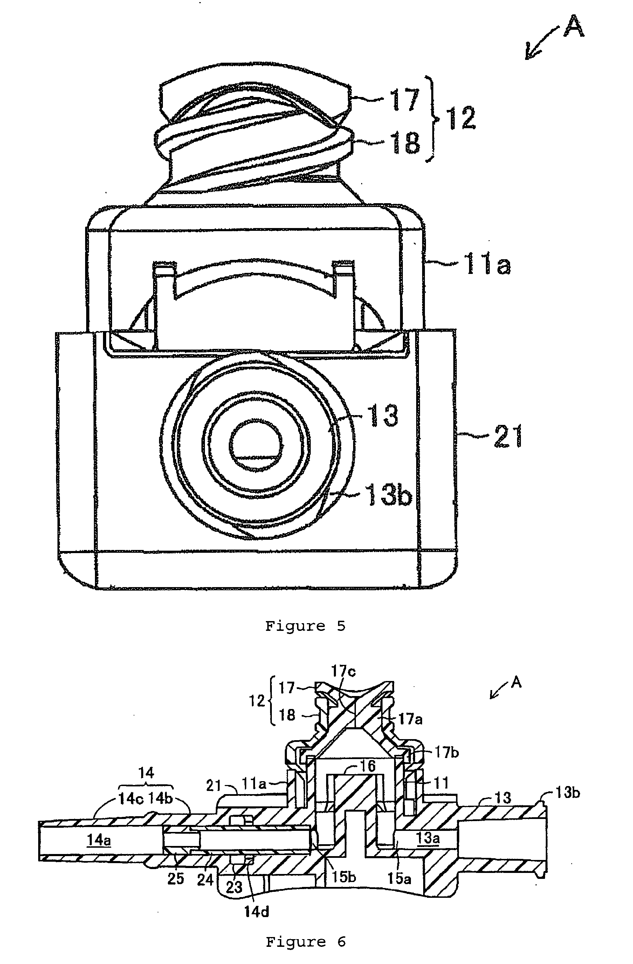

The fluid mixture delivery instrument pertaining to Mode of Embodiment 1 of the present invention will be described below in detail with reference to the figures. Figures 1 to 5 show a fluid mixture delivery instrument A pertaining to this mode of embodiment,
where said fluid mixture delivery instrument A is configured by a fluid mixture delivery instrument main body 10 and an open/close operating part 20. The fluid mixture delivery instrument main body 10 is configured by a chamber part 11 which is formed with a roughly cylindrical shape that is short in the axial direction and which is arranged vertically in said axial direction (see Figures 6 to 8), a merging pipe 12 which is provided on top of the chamber part 11, and an upstream pipe 13 and downstream pipe 14 which extend to both lateral sides of the chamber part 11 along the same axis at an angle of 180°.

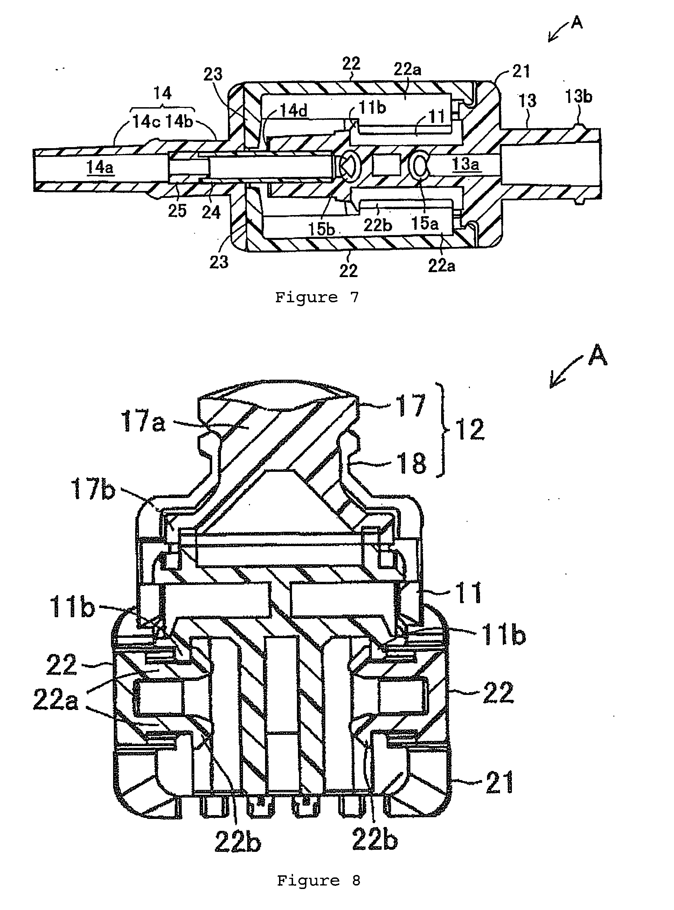

The chamber part 11 is formed with a closed lower surface configured by a recessed part, and roughly in the shape of a cylinder which has a bottom and an open upper end. Then, a frame-like attachment part 21 which forms an approximate square in which the width at the centre is smaller than the width at both lateral ends when seen from above, and which is provided with opening parts at the front and rear, is formed on the outer periphery of the chamber part 11. Furthermore, as shown in Figures 6 and 7, respective through-holes 15a, 15b are formed in a laterallyfacing portion somewhat lower down than the centre in the axial direction of the chamber part 11. An upstream pipe 13 is provided in a portion corresponding to the through-hole 15a in the chamber part 11, while a flow channel 13a formed inside the upstream pipe 13 and the inside of the chamber part 11 are linked in communication, via the through-hole 15a.

A downstream pipe 14 is provided in a portion corresponding to the through-hole 15b in the chamber part 11, while the inside of the chamber part 11 and a flow channel 14a formed inside the downstream pipe 14 are linked in communication via the through-hole 15b. Said chamber part 11, upstream pipe 13, downstream pipe 14 and attachment part 21 are integrally formed. Furthermore, the flow channel 13a formed inside the upstream pipe 13 is configured by a stepped hole part in which the portion linking in communication with the through-hole 15a on the chamber part 11 side is formed with the same diameter as the through-hole 15a, and in which the opening side portion has a larger diameter than the chamber part 11 side portion.

The opening side portion of the flow channel 13a is then formed with a tapering shape in which the diameter on the chamber part 11 side is smaller, with the diameter becoming gradually larger nearer the opening. In other words, the opening side portion of the upstream pipe 13 is formed in the shape of a female luer. A linking screw part 13b is then formed on the outer periphery of the opening part of the upstream pipe 13. The downstream pipe 14 is configured by a base end part 14b which lies on the chamber part 11 side, and a male luer part 14c which lies at the tip end of the downstream pipe and is narrower than the base end part 14b. Furthermore, the male luer part 14c tapers off, becoming gradually narrower at the tip end side than at the base end part 14b side.

A cylindrical part to be engaged 11a which maintains a spacing from the cylindrical opening part at the top of the chamber part 11 is formed on the periphery of the cylindrical opening part which is located at the top of the chamber part 11. A vertical wall 16 extending upwards from approximately the centre in the lateral direction (the lateral direction in Figures 6 and 7) of the inner bottom surface of the chamber part 11 is provided inside the chamber part 11. Said vertical wall 16 is provided so as to cut across the central portion in the width direction orthogonal to the direction linking the flow channel 13a portion and the flow channel 14a portion inside the chamber part 11, and its upper end part extends to the vicinity of the upper end of the chamber part 11.

The merging pipe 12 is configured by a rubber stopper 17 made of natural rubber or synthetic rubber, which is fitted to the upper end part of the cylindrical opening part of the chamber part 11, and a cap member 18 fitted to the outer periphery of the rubber stopper 17 so as to fix the rubber stopper 17 to the upper end part of the cylindrical opening part of the chamber part 11. The rubber stopper 17 is configured by a thick, disc-shaped stopper main body 17a, and a pair of approximately strip-shaped fixing pieces 17b which extend from both lower end sides of the stopper main body 17a. Said rubber stopper 17 is fixed by placing both fixing pieces 17b on the outer periphery of the upper end part of the cylindrical opening part of the chamber part 11, and then fitting the cap member 18 to the outer periphery thereof.

The cap member 18 is configured by a two-stage cylindrical body comprising a large-diameter lower part which engages with the chamber part 11, and a small-diameter upper part. The cap member 18 is then fixed to the chamber part 11 by inserting the lower part of the large-diameter portion between the cylindrical opening part of the chamber part 11 and the part to be engaged 11a, in a state in which the fixing pieces 17b are pressured onto the cylindrical opening part of the chamber part 11 by the upper part of the large-diameter portion. Furthermore, the upper end of the narrow portion at the top of the cap member 18 is open, and the rubber stopper 17 is fixed inside the cap member 18, in a state in which the upper part of the rubber stopper 17 is prevented from protruding from the upper end and in which the rubber stopper 17 is prevented from entering the cap member 18. Furthermore, a slit 17c for linking the inside of the chamber part 11 with the outside is provided in the stopper main body 17a of said rubber stopper 17.

Said slit 17c is normally in a state in which it is closed by the elasticity of the rubber stopper 17. Then, when the inside of the chamber part 11 and the outside are linked in communication, a flow channel can be formed via a connector or the like (not depicted), by inserting said connector or the like into the slit 17c in the rubber stopper 17. Said connector consists of a male luer, for example, which has a flow channel formed inside, and the connector and the inside of the chamber part 11 can be linked in communication by inserting the male luer part into the slit 17c of the rubber stopper 17. Furthermore, the area between the male luer and the peripheral surface of the slit 17c is in a state of close contact at this time, due to the elasticity of the rubber stopper 17.

The open/close operating part 20 is configured by: the attachment part 21 which is formed on the outer periphery of the chamber part 11; pairs of operating parts consisting of rocking pieces 22 which act as the downstream rocking pieces of the present invention, and pressure parts 23 which act as the downstream pressure parts of the present invention, these parts being linked to the attachment part 21; a flexible tube 24 which acts as the downstream flexible tube of the present invention and is arranged inside the downstream pipe 14; and a fixing member 25 which fixes the tube 24 to the inside of the downstream pipe 14. The attachment part 21 is configured by an approximately square box-shaped body in which the longitudinal width is somewhat greater than the height. Square-shaped openings which are laterally long are formed at the front and rear of said square box-shaped body. Furthermore, the bottom surface of the attachment part 21 is such that the centre in the lateral direction is formed as a curving surface which curves upwards, and space parts (recessed parts) are formed at prescribed portions thereof.

Said space part is formed not only as the bottom surface portion of the attachment part 21, but also as a continuation of the lower part of the chamber part 11, and part of it extends from the lower surface of the vertical wall 16 along the inside. Providing this space makes it possible to prevent shrinkage holes being produced and to use only a small amount of moulding material when the attachment part 21 is moulded. Furthermore, the base end parts of the rocking pieces 22 are respectively linked to the edge part on the upstream pipe 13 side at both the front and rear openings of the attachment part 21. Both rocking pieces 22 are pivotably mounted between the peripheral edge part of the opening of the attachment part 21 and the inside of the attachment part 21, with the base end part as the centre, in a state in which the base end part is connected to the edge part of the opening.

The pressure parts 23 are integrally formed with the rocking pieces 22 at the tip end part of the respective rocking pieces 22, and they extend to the inside of the attachment part 21, orthogonal to the rocking pieces 22. Furthermore, an insertion hole 14d penetrating from the outer peripheral surface to the inner peripheral surface of the downstream pipe 14 is formed at a portion opposite the tip end of both pressure parts 23 for the downstream pipe 14, and the tip end part of both pressure parts 23 lies inside the insertion hole 14d. When no pressure is applied to the rocking pieces 22, the tip end part of the pressure parts 23 lies in the region of the inner peripheral surface of the downstream pipe 14, but when pressure is applied to the rocking pieces 22, the tip end part of the pressure parts 23 progresses into the downstream pipe 14.

Furthermore, plate-shaped reinforcing ribs 22a extending from the base end side of the rocking pieces 22 to the pressure parts 23 are formed on both sides in the width direction, vertically on the inner surface of the rocking pieces 22. The width of the plate surface of the plate-shaped ribs 22a at the portion lying on the base end side of the rocking pieces 22 (the width in the longitudinal direction) is formed to be greater than the width of the plate surface at the portion lying on the pressure part 23 side, and a stepped part is formed at the boundary between them. Furthermore, a small space is provided between the large-width portion of the plate-shaped ribs 22a and the outer peripheral surface of the chamber part 11, and a space which is larger than the space between the large-width portion and the chamber part 11 is provided between the narrow-width portion of the plate-shaped ribs 22a and the outer peripheral surface of the downstream pipe 14.

A hook-shaped engagement part 22b is then formed at the tip end of the large-width portion of the plate-shaped ribs 22a, and a part to be engaged 11b which engages with the engagement part 22b and prevents the rocking pieces 22 from protruding outside the attachment part 21 is formed on the outer peripheral surface of the chamber part 11. Consequently, the rocking pieces 22 can bend towards the inside of the attachment part 21, but they cannot protrude outside of the attachment part 21. Furthermore, the portion opposite the stepped part of the plate-shaped ribs 22a at the part to be engaged 11b of the chamber part 11 is formed as a guide part which can slidably engage with said stepped part.

The tube 24 is configured by a cylindrical body made of soft, flexible plastic. Said tube 24 is arranged in a portion from roughly the centre in the lateral direction of the downstream pipe 14 inside the flow channel 14a to the chamber part 11 in a state in which it is in close contact with the inner peripheral surface of the downstream pipe 14, and the downstream end is fixed to the inner peripheral surface of the downstream pipe 14 by means of the fixing member 25. The fixing member 25 is configured by a two-stage cylindrical body in which the base end side is of greater diameter than the tip end side, and it is arranged inside the downstream pipe 14 in a state in which the large-diameter base end side lies on the downstream side, and the narrow-diameter tip end side lies on the upstream side. In other words, the fixing member 25 fixes the tube 24 by means of the fact that the tip end side portion is inserted into the downstream end side portion of the tube 24, and the downstream end side portion of the tube is held between the outer peripheral surface of the tip end side portion and the inner peripheral surface of the downstream pipe 14. The fixing member 25 is then fixed inside the downstream pipe 14 by means of the fact that the base end side portion is pressured by contact with the inner peripheral surface of the downstream pipe 14.

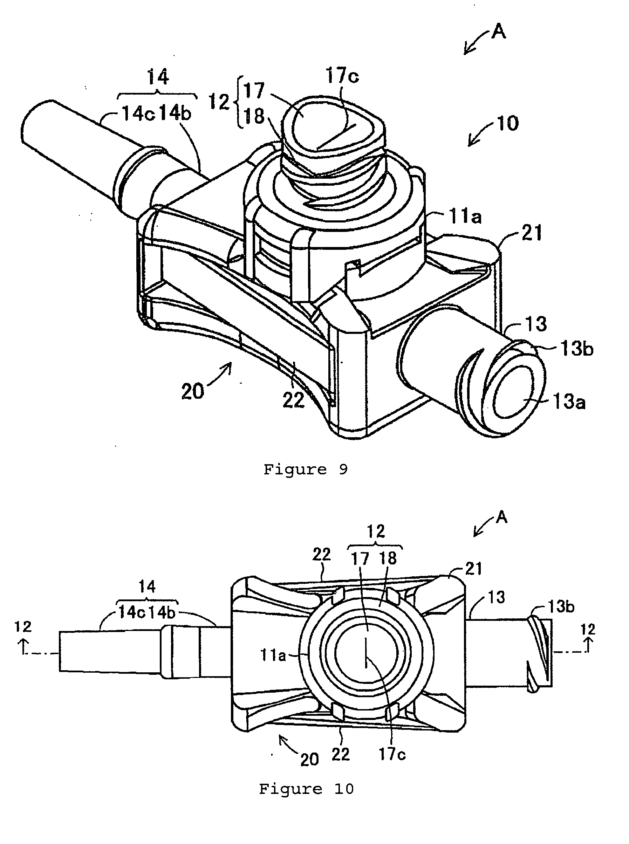

Consequently, as shown in Figure 9, when pressure is applied to the respective tip end sides of the rocking pieces 22 inside the attachment part 21, the tip end side portions of both rocking pieces 22 and the pressure parts 23 come closer together, achieving the state shown in Figures 10 to 14. By virtue of this, the portions 24a in the tube 24 which are pinched by both pressure parts 23 come into pressure-contact, and the flow channel between the flow channel 14a of the downstream pipe 14 and the inside of the chamber part 11 is closed off. When both of these rocking pieces 22 are pressure-operated, the stepped part of the plate-shaped ribs 22a is guided by the guide part of the part to be engaged 11b in the chamber part 11, and therefore the rocking pieces 22 and the pressure parts 23 move smoothly.

Furthermore, when the force whereby both rocking pieces 22 are pressured inside the attachment part 21 is released, the tube 24 returns to its original cylindrical shape, and the state shown in Figures 6 to 8 is achieved, in which the tip end side portion of both rocking pieces 22 and both pressure parts 23 are separated from each other by the return force of said tube 24. Then, when the tube 24 returns to its original cylindrical shape, the inside of the chamber part 11 and the flow channel 14a of the downstream pipe 14 are linked in communication. In this case, when a drug solution or the like is supplied from the upstream pipe 13 side to the chamber part 11 side, said drug solution or the like flows from the flow channel 13a into the chamber part 11 and surmounts the vertical wall 16, after which it flows from inside the chamber part 11 into the flow channel 14a of the downstream pipe 14. At this time, the vertical wall 16 prevents return flow of the drug solution or the like, and also air retention inside the chamber part 11 is prevented by causing the drug solution or the like to pass through inside the upper part of the chamber part 11.

With this configuration, when a certain drug solution is supplied inside the body of a patient (not depicted), a female luer part which constitutes the rear end part of a transfusion tube (not depicted) to which an indwelling needle which pierces the patient in order to remain there is connected, is connected to the downstream pipe 14. Furthermore, a male luer part provided at the tip end part of a transfusion tube which extends from a container or the like housing the drug solution to be supplied to the patient, is connected to the upstream pipe 13. Then, with the indwelling needle having pierced the patient's body and remaining therein, the drug solution can be supplied to the patient by sending the drug solution in the container or the like towards the patient. Furthermore, when another drug solution is to be supplied to the patient in addition to the drug solution supplied from the container or the like, the other drug solution is delivered from the merging pipe 12 into the chamber part 11, via the transfusion tube which is connected to the connector.

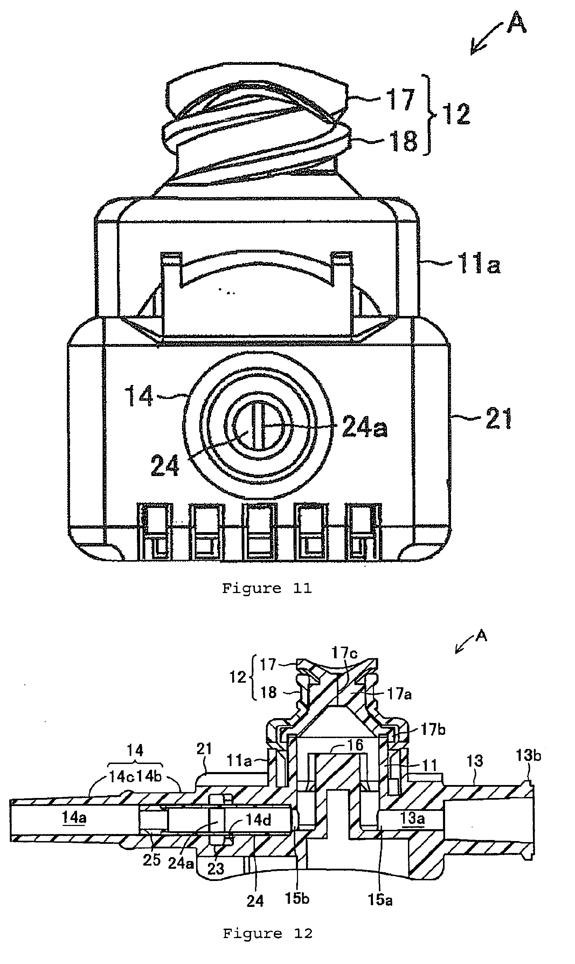

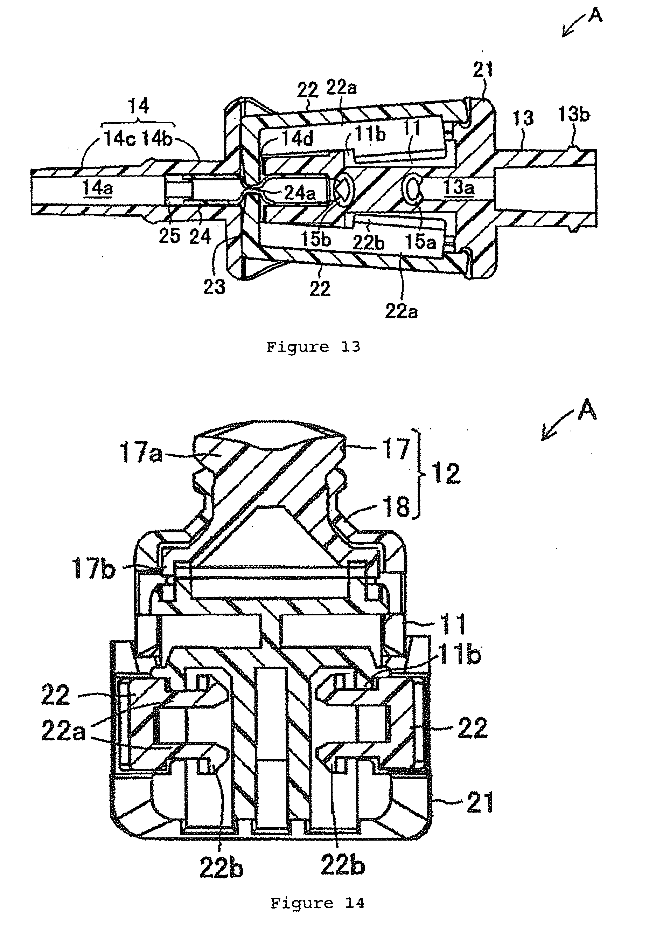

In this case, when the connector is inserted into the slit 17c in the rubber stopper 17 of the merging tube 12, the connector and the downstream pipe 14 are linked in communication via the flow channel inside the chamber part 11, and therefore the drug solution can be supplied to the patient by delivery of said drug solution from the connector side. Furthermore, if the drug solution which is supplied from the merging pipe 12 is to be used when mixed in a fixed amount with the drug solution which is supplied from the upstream pipe 13, the tip end sides of the rocking pieces 22 are pushed inside the attachment part 21, and the tube 24 is closed off, as shown in Figures 12 and 13. In this state, when the drug solution is delivered from the merging pipe 12 side into the chamber part 11, said drug solution is prevented from flowing to the downstream pipe 14 side, and flows to the upstream pipe 13 side.

Then, the supply of the drug solution is stopped when the amount of said drug solution supplied from the merging pipe 12 has reached the set amount, and the connector is removed from the rubber stopper 17. By virtue of this, the merging pipe 12 is closed off. Then, if two types of drug solutions are mixed at the upstream side of the upstream pipe 13, pressure on the rocking pieces 22 is released. By virtue of this, the closed off portion 24a of the tube 24 opens, and the drug solution mixture comprising two liquids is supplied to the patient from the container or the like. It should be noted that a small amount of the drug solution or the like should be ejected from the tip end of the indwelling needle before said indwelling needle pierces the patient's body and remains therein. By virtue of this, air inside the flow channel is ejected to the outside together with the drug solution. Furthermore, when the drug solution which passes from the upstream pipe 13 side into the chamber part 11 and flows to the downstream pipe 14 side passes inside the chamber part 11, it surmounts the vertical wall 16 and passes through the upper part of the chamber part 11, and therefore air retention inside the chamber part 11 is prevented.

In this way, the fluid mixture delivery instrument A pertaining to this mode of embodiment is configured by the chamber part 11, the fluid mixture delivery instrument main body 10 which comprises the upstream pipe 13, downstream pipe 14 and merging pipe 12, all of which link in communication with the chamber part 11, and the open/close operating part 20. Then, the open/close operating part 20 is configured by the tube 24 which is arranged inside the downstream pipe 14, and the pressure parts 23 and rocking pieces 22 etc. for closing off the tube 24. Consequently, it is possible to send a drug solution or the like from the upstream pipe 13 to the downstream pipe 14, via the chamber part 11, and it is also possible to send a fluid such as another drug solution from the merging pipe 12 to the downstream pipe 14, via the chamber part 11.

Furthermore, by closing off the tube 24 by the pressure-operation of the rocking pieces 22, it is possible to send a drug solution supplied from the merging pipe 12 to the upstream side of the upstream pipe 13, and to mix this with a drug solution which is supplied from the upstream pipe 13. Then, after the two types of drug solution have been mixed, the pressure on the rocking pieces 22 is released to link both ends of the tube 24 in communication, whereby the two mixed liquids can be sent from the upstream pipe 13 to the downstream pipe 14 via the chamber part 11. This makes it possible to mix two types of drug solutions and then to supply them to the patient's body. Furthermore, the pressure parts 23 are provided at one end of the elongate rocking pieces 22, the other end of which is supported outside the fluid mixture delivery instrument main body 10, and therefore when the pressure parts 23 are pressure-operated, this makes it possible for the fingers to make contact with the rocking pieces 22 instead of the pressure parts 23, simplifying the pressure operation.

Furthermore, the operation at this time is simple to achieve simply by pinching and pushing the pair of rocking pieces 22 between the thumb and forefinger. The operation to link both ends of the closed-off tube 24 in communication is also extremely simple to achieve, simply by releasing the pressure-operation of the pair of rocking pieces 22. Furthermore, the tube 24 is fixed to the inner peripheral surface of the downstream pipe 14 by the cylindrical fixing member 25, and therefore the tube 24 can be securely fixed to the inner peripheral surface of the upstream pipe 13, and it can be maintained in a state in which it is fixed to the inner peripheral surface of the downstream pipe 14, even if the tube 24 is compressed by pressure on the pressure parts 23.

### (Mode of Embodiment 2)

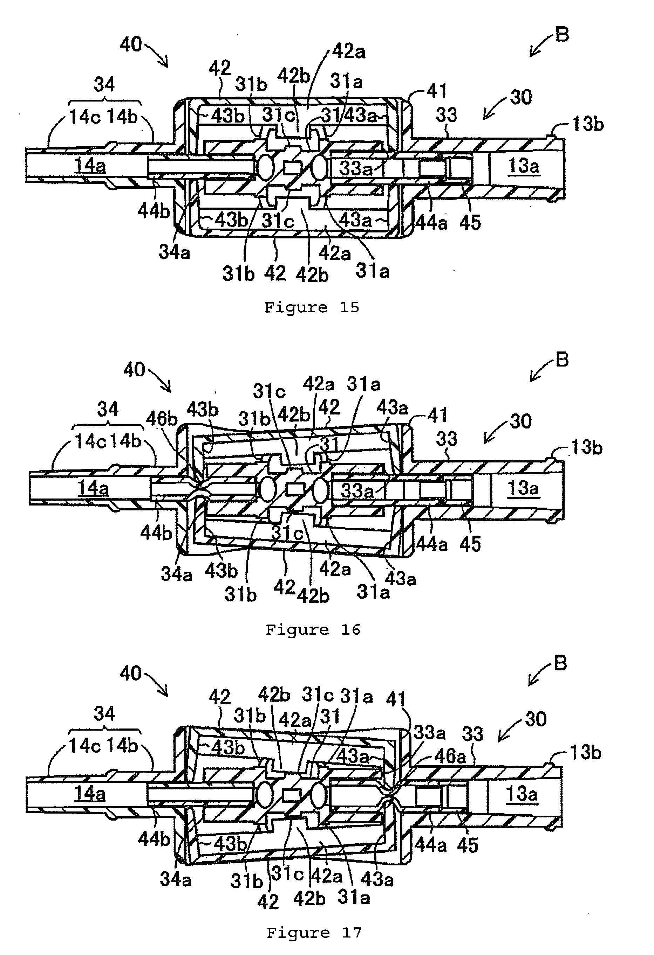

Figures 15 to 17 show the main parts of a fluid mixture delivery instrument B pertaining to Mode of Embodiment 2 of the present invention. With this fluid mixture delivery instrument B, the open/close operating part 40 is configured by: an attachment part 41 which is formed on the outer peripheral portion of a chamber part 31 in a fluid mixture delivery instrument main body 30; a pair of rocking pieces 42 which are respectively attached to both the front and rear sides of the chamber part 31; upstream pressure parts 43a which are provided at one end (the upstream end at the right-hand end in Figures 15 to 17) in the lateral direction of the pair of respective rocking pieces 42; downstream pressure parts 43b which are provided at the other end (the downstream end at the left-hand end in Figures 15 to 17) in the lateral direction of the pair of respective rocking pieces 42; an upstream flexible tube 44a which is arranged inside an upstream pipe 33; a downstream flexible tube 44b which is arranged inside a downstream pipe 34; and a fixing member 45 which fixes the upstream flexible tube 44a inside the upstream pipe 33.

Plate-shaped reinforcing ribs 42a extending from one end to the other of the rocking pieces 42 are formed on both sides in the width direction, vertically on the inner surface of the pair of rocking pieces 42. Supported parts 42b which project towards the chamber part 31 side are then formed in the centre in the lateral direction on the inner surface of the plate-shaped ribs 42a. Furthermore, support parts 31a, 31b for supporting the rocking pieces 42 in such a way that they can rock are formed at portions corresponding to both lateral ends of the supported parts 42b on the outer peripheral surface of the chamber part 31, in a state in which the supported parts 42b are held between them. Furthermore, protrusions 31c are formed at portions opposite the central part of the supported parts 42b on the side surface of the chamber part 31, and the rocking pieces 42 can rock with these protruding parts 31c as a fulcrum.

The support parts 31a, 31b are held in a state in which they retain the supported parts 42b, and the rocking pieces 42 are prevented from protruding outside the attachment part 41. Consequently, both ends of the rocking pieces 42 can respectively move forward inside the attachment part 41, by pivoting with the protrusions 31c as a fulcrum with the central part of the supported parts 42b making contact with the protrusions 31c, but they cannot protrude outside the attachment part 41. The upstream pressure parts 43a are integrally formed with the rocking pieces 42, at one end of the rocking pieces 42, so as to be orthogonal to the respective rocking pieces 42 and to extend inside the attachment part 41, and the downstream pressure parts 43b are integrally formed with the rocking pieces 42, at the other end of the rocking pieces 42, so as to be orthogonal to the respective rocking pieces 42 and to extend inside the attachment part 41.

Furthermore, an insertion hole 33a which passes from the outer peripheral surface to the inner peripheral surface of the upstream pipe 33 is formed at a portion opposite the tip end of both upstream pressure parts 43a for the upstream pipe 33, and the tip end part of both upstream pressure parts 43a lies inside the insertion hole 33a. When no pressure is applied to a portion at one end of the rocking pieces 42, the tip end part of the upstream pressure parts 43a lies in the region of the inner peripheral surface of the upstream pipe 33, but when pressure is applied to a portion at one end of the rocking pieces 42, the tip end part of the upstream pressure parts 43a progresses into the upstream pipe 33. Furthermore, an insertion hole 34a which passes from the outer peripheral surface to the inner peripheral surface of the downstream pipe 34 is also formed at a portion opposite the tip end of both downstream pressure parts 43b for the downstream pipe 34, and the tip end part of both downstream pressure parts 43b lies inside the insertion hole 34a.

When no pressure is applied to the other end portion of the rocking pieces 42, the tip end part of the downstream pressure parts 43b lies in the region of the inner peripheral surface of the downstream pipe 34, but when pressure is applied to the other end portion of the rocking pieces 42, the tip end part of the downstream pressure parts 43b progresses into the downstream pipe 34. The upstream flexible tube 44a and the downstream flexible tube 44b are configured by a cylindrical body made of soft flexible plastic, in the same way as the abovementioned tube 24. The upstream flexible tube 44a is then arranged at a portion running from roughly the centre in the lateral direction inside the upstream pipe 33 to the chamber part 31, in a state in which it is in close contact with the inner peripheral surface of the upstream pipe 33, and the upstream end is fixed to the inner peripheral surface of the upstream pipe 33 by means of a fixing member 45.

The fixing member 45 is configured by a member comprising a two-stage cylindrical body which is similar to the abovementioned fixing member 25, and it is arranged inside the upstream pipe 33 in a state in which the large-diameter base end side lies on the upstream side, and the narrow-diameter tip end side lies on the downstream side. Furthermore, the downstream flexible tube 44b is arranged in a portion running from roughly the centre in the lateral direction inside the downstream pipe 34 to the chamber part 31, in a state in which it is in close contact with the inner peripheral surface of the downstream pipe 14. The other structural components of this fluid mixture delivery instrument B are the same as those of the fluid mixture delivery instrument A described above. Accordingly, the same components carry the same reference numbers, and a description thereof will be omitted.

By virtue of this configuration, as shown in Figure 15, when pressure is applied to the respective portions on the downstream pressure parts 43b side of the rocking pieces 42 inside the attachment part 41, the downstream pressure parts 43b assume a state in which they come closer together. Because of this, the portions 46b in the downstream flexible tube 44b which are pinched by both downstream pressure parts 43b come into pressure-contact, and the flow channel between the flow channel 34a of the downstream pipe 34 and the inside of the chamber part 31 is closed off. Furthermore, as shown in Figure 16, when pressure is applied to the respective portions on the upstream pressure parts 43a side of the rocking pieces 42 inside the attachment part 41, the upstream pressure parts 43a assume a state in which they come closer together.

Because of this, the portions 46a in the upstream flexible tube 44a which are pinched by both upstream pressure parts 43a come into pressure-contact, and the flow channel between the flow channel 13a of the upstream pipe 33 and the inside of the chamber part 31 is closed off. Furthermore, when the force is released from the state in which one of the lateral sides of both of the rocking pieces 42 is being pressured inside the attachment part 41, the upstream flexible tube 44a or the downstream flexible tube 44b which was closed off returns to its original cylindrical shape, and the state shown in Figure 15 is achieved. Then, in this state, the area between the flow channel 13a of the upstream pipe 33 and the chamber part 31, and the area between the chamber part 31 and flow channel 14a of the downstream pipe 34 are linked in communication.

According to the fluid mixture delivery instrument B pertaining to this mode of embodiment, a drug solution or the like which is supplied form the merging pipe 12 can be sent to the upstream side of the upstream pipe 33, and mixed with a drug solution or the like which is supplied from the upstream pipe 33, by pressuring the portion on the downstream pressure parts 43b side of the rocking pieces 42 to close off the downstream flexible tube 44b. Furthermore, it is possible to stop or reopen the supply of one of the two types of drug solutions or the like which is sent from the upstream pipe 33 to the downstream pipe 34, via the chamber part 31, by applying pressure to or releasing the pressure on the portion at the upstream pressure parts 43a side of the rocking pieces 42. The other operational effects of this fluid mixture delivery instrument B are the same as those of the fluid mixture delivery instrument A described above.

Furthermore, the fluid mixture delivery instrument pertaining to the present invention is not limited to the modes of embodiment described above, and appropriate modifications may be implemented. For example, in the modes of embodiment described above, the rubber stopper 17 is provided with a slit 17c, and a connector is inserted into the slit 17c, whereby the connector is connected to the merging pipe 12, but the male luer part of a syringe or an injection needle may be inserted instead of the connector. When an injection needle is inserted, there is no need to provide the slit 17c in the rubber stopper 17. Furthermore, in Mode of Embodiment 1 described above, the pressure parts 23 are provided at the tip end of the rocking pieces 22 for the pressure-operation of the rocking pieces 22, but the rocking pieces 22 may be dispensed with, and the pressure parts 23 may be pressure-operated directly.

This means that it is possible to reduce the size of the attachment part 21. In addition, it is also possible to implement appropriate modifications to the shape and material etc. of the other components which make up the fluid mixture delivery instrument. Furthermore, in Mode of Embodiment 2 described above, the downstream flexible tube 44b is attached to the inner peripheral surface of the downstream pipe 34 without using a fixing member, but said downstream flexible tube 44b can also preferably be fixed to the inner peripheral surface of the downstream pipe 34 using a fixing member similar to the fixing member 45. In this case, the fixing member is preferably arranged at the downstream end of the downstream flexible tube 44b.

### [Brief Description of the Figures]

[Figure 1] is an oblique view showing the fluid mixture delivery instrument pertaining to Mode of Embodiment 1 of the present invention;
[Figure 2] is a plan view of the fluid mixture delivery instrument;
[Figure 3] is a front view of the fluid mixture delivery instrument;
[Figure 4] is a left-side view of the fluid mixture delivery instrument;
[Figure 5] is a right-side view of the fluid mixture delivery instrument;
[Figure 6] is a view in cross section along 6-6 in Figure 2;
[Figure 7] is a view in cross section along 7-7 in Figure 3;
[Figure 8] is a view in cross section along 8-8 in Figure 3;
[Figure 9] is an oblique view showing the fluid mixture delivery instrument in a state in which the rocking pieces have been pressure-operated;
[Figure 10] is a plan view of the fluid mixture delivery instrument in Figure 9;
[Figure 11] is a left-side view of the fluid mixture delivery instrument in Figure 9;
[Figure 12] is a view in cross section showing a state seen from the front of the fluid mixture delivery instrument in Figure 9;
[Figure 13] is a view in cross section showing a state seen from above the fluid mixture delivery instrument in Figure 9;
[Figure 14] is a view in cross section showing a state seen from the side of the fluid mixture delivery instrument in Figure 9;
[Figure 15] is a view in cross section showing a state seen from above the fluid mixture delivery instrument pertaining to Mode of Embodiment 2 of the present invention;
[Figure 16] is a view in cross section showing a state in which the downstream pipe of the fluid mixture delivery instrument shown in Figure 15 is closed off; and
[Figure 17] is a view in cross section showing a state in which the upstream pipe of the fluid mixture delivery instrument shown in Figure 15 is closed off.

### [Explanation of Symbols]

10, 30...fluid mixture delivery instrument; 11, 31...chamber part; 12...merging pipe; 13, 33...upstream pipe; 14, 34...downstream pipe; 14d, 33a, 34a...insertion hole; 22, 42...rocking piece; 23...pressure part; 24...tube; 25, 45...fixing member; 43a...upstream pressure part; 43b...downstream pressure part; 44a...upstream flexible tube; 44b...downstream flexible tube; A, B...fluid mixture delivery instrument.

## Claims

1. Fluid mixture delivery instrument **characterized in that** it comprises:
a fluid mixture delivery instrument main body provided with an upstream pipe for sending fluid to a chamber part, a downstream pipe to which the abovementioned fluid is sent from the abovementioned chamber part, and
a merging pipe for sending another fluid to the abovementioned chamber part;
a downstream flexible tube which is arranged inside the abovementioned downstream pipe, and inside which the abovementioned fluid sent from the abovementioned chamber part to the abovementioned downstream pipe flows; and
a downstream pressure part which extends from the outside to the inside of the abovementioned downstream pipe, and which closes off the abovementioned downstream flexible tube by means of pressure applied to the inside of the abovementioned downstream pipe.

2. Fluid mixture delivery instrument according to Claim 1, in which two abovementioned downstream pressure parts are provided, one on either side of the abovementioned downstream flexible tube.

3. Fluid mixture delivery instrument according to Claim 1 or 2, in which the abovementioned downstream pressure part is provided at one end of a downstream rocking piece, the other end of which is supported outside the abovementioned fluid mixture delivery instrument main body.

4. Fluid mixture delivery instrument according to any one of Claims 1 to 3, in which the upstream end or the downstream end of the abovementioned downstream flexible tube is fixed to the inner peripheral surface of the abovementioned downstream pipe or to the inside of the abovementioned chamber part, by a cylindrical fixing member.

5. Fluid mixture delivery instrument according to any one of Claims 1 to 4, comprising:
an upstream flexible tube which is arranged inside the abovementioned upstream pipe, and inside which the abovementioned fluid sent from the abovementioned upstream pipe to the abovementioned chamber part flows; and
an upstream pressure part which extends from the outside to the inside of the abovementioned upstream pipe, and which closes off the abovementioned upstream flexible tube by means of pressure applied to the inside of the abovementioned upstream pipe.

6. Fluid mixture delivery instrument according to Claim 5, in which two abovementioned upstream pressure parts are provided, one on either side of the abovementioned upstream flexible tube.

7. Fluid mixture delivery instrument according to Claim 5 or 6, in which the abovementioned upstream pressure part is provided at one end of an upstream rocking piece, the other end of which is supported outside the abovementioned fluid mixture delivery instrument main body.

8. Fluid mixture delivery instrument according to any one of Claims 5 to 7, in which the upstream end of the abovementioned upstream flexible tube is fixed to the inner peripheral surface of the abovementioned upstream pipe by a cylindrical fixing member.

9. Fluid mixture delivery instrument **characterized in that** it comprises:
a fluid mixture delivery instrument main body provided with an upstream pipe for sending fluid to a chamber part, a downstream pipe to which the abovementioned fluid is sent from the abovementioned chamber part, and
a merging pipe for sending another fluid to the abovementioned chamber part;
a downstream flexible tube which is arranged inside the abovementioned downstream pipe, and inside which the abovementioned fluid sent from the abovementioned chamber part to the abovementioned downstream pipe flows;
an upstream flexible tube which is arranged inside the abovementioned upstream pipe, and inside which the abovementioned fluid sent from the abovementioned upstream pipe to the abovementioned chamber part flows; a downstream pressure part which extends from the outside to the inside of the abovementioned downstream pipe, and which closes off the abovementioned downstream flexible tube by means of pressure applied to the inside of the abovementioned downstream pipe;
an upstream pressure part which extends from the outside to the inside of the abovementioned upstream pipe, and which closes off the abovementioned upstream flexible tube by means of pressure applied to the
inside of the abovementioned upstream pipe; and
a rocking piece at both ends of which provision is made for the abovementioned downstream pressure part and the abovementioned upstream pressure part, and the central part of which is supported outside the abovementioned fluid mixture delivery instrument main body so as to be able to rock back and forth.

## Amended claims

### Amended claims in accordance with Rule 137(2) EPC.

**1.** Fluid mixture delivery instrument (A, B) comprising:
a fluid mixture delivery instrument main body (10, 40) provided with an upstream pipe (13) for sending fluid to a chamber part (11), a downstream pipe (14) to which the fluid is sent from the chamber part, and a merging pipe (12) for sending another fluid to the chamber part;
a downstream flexible tube (24) which is arranged inside the downstream pipe, and through which the fluid sent from the chamber part to the downstream pipe flows; and
a downstream pressure part (23) which extends from the outside to the inside of the downstream pipe, and which closes off the downstream flexible tube by means of pressure applied to the outside of the-downstream flexible tube,
**characterized** that the downstream pressure part is provided at one end of a rocking piece (22), and that when said downstream flexible tube is in a closed state, said upstream pipe and said merging pipe are in fluid flow communication such that fluid supplied from the merging pipe passes to an upstream side of said upstream pipe.

**2.** Fluid mixture delivery instrument according to claim 1, in which two downstream pressure parts (23) are provided, one on either side of the downstream flexible tube (24).

**3.** Fluid mixture delivery instrument according to claim 1 or 2, in which other end of the rocking piece (23) is supported outside the fluid mixture delivery instrument main body (10).

**4.** Fluid mixture delivery instrument according to any of the claims 1 to 3, in which the upstream end or the downstream end of the downstream flexible tube (24) is fixed to the inner peripheral surface of the chamber part by a cylindrical fixing member (25).

**5.** Fluid mixture delivery instrument according to any of the claims 1 to 4, further comprising:
an upstream flexible tube (44a) which is arranged inside the upstream pipe, and inside which the fluid sent from the upstream pipe to the chamber part flows; and
an upstream pressure part (43a) which extends from the outside to the inside of the upstream pipe, and which closes off the upstream flexible tube by means of pressure applied to the-upstream flexible tube (44a).

**6.** Fluid mixture delivery instrument according to claim 5, in which two upstream pressure parts (43a) are provided, one on either side of the upstream flexible tube (44a).

**7.** Fluid mixture delivery instrument according to claim 5 or 6, in which the upstream pressure part (43a) is provided at the other end of the rocking piece (42).

**8.** Fluid mixture delivery instrument according to any of the claims 5 to 7, in which the upstream end of the upstream flexible tube (44a) is fixed to the inner peripheral surface of the upstream pipe by a cylindrical fixing member (45).
